# EUROPÄISCHE PATENTSCHRIFT

(11) **EP 2 405 813 B1**
(45) Veröffentlichungstag und Bekanntmachung des Hinweises auf die Patenterteilung: **08.11.2017**
(21) Anmeldenummer: 10708212.5
(22) Anmeldetag: 12.03.2010
(51) Int. Cl.: A61B 5/107, B68G 15/00, B68F 3/00, G01B 5/207, B43L 13/20, A61B 5/103

(54) **MESSVORRICHTUNG UND VERFAHREN ZUR BESTIMMUNG EINES OBERFLÄCHENVERLAUFS AN EINEM MENSCHEN ODER TIER**
MEASURING DEVICE AND METHOD FOR DETERMINING A SURFACE CONTOUR ON A HUMAN OR ANIMAL
DISPOSITIF DE MESURE ET PROCÉDÉ POUR DÉTERMINER UNE SURFACE D'UN HOMME OU D'UN ANIMAL

(30) Priorität: 12.03.2009 DE 102009012467; 14.04.2009 DE 102009016970
(43) Veröffentlichungstag der Anmeldung: 18.01.2012
(73) Patentinhaber: Rieser, Christoph, 56593 Obersteinebach (DE)
(72) Erfinder: Rieser, Christoph, 56593 Obersteinebach (DE)
(74) Vertreter: Patentanwälte Bauer Vorberg Kayser
(86) Internationale Anmeldenummer: PCT/EP2010/053198
(87) Internationale Veröffentlichungsnummer: WO 2010/103108

(56) Entgegenhaltungen:
- DE-C- 100 464
- US-A- 1 129 081
- US-A- 5 188 121
- US-A1- 2003 083 596
- US-B1- 6 334 262

## Beschreibung

Die Erfindung betrifft eine mehrere drehgelenkig verbundene Kettenglieder umfassende Messvorrichtung zur Bestimmung eines Oberflächenverlaufs an einem Menschen oder Tier.

Solche Messvorrichtungen sind bekannt. Beispielsweise ist aus der Druckschrift DE 100 464 A eine gelenkige Maßlehre zum Bestimmen der Maße und Profile von Tierkörpern bekannt, wobei die Maßlehre zwei von einem Mittelteil nach rechts und links sich erstreckende, vielgliedrige, gelenkige Bänder aufweist, deren einzelne Glieder mit wellenartigen Erhöhungen ineinander greifen und dadurch in der gegebenen Richtung feststehen. Nachteilig an dieser Maßlehre ist, dass einerseits lediglich die Vermessung eines einzigen Querschnitts des Tierkörpers möglich ist, da mittels der Maßlehre lediglich der Umfang des Tierkörpers in einer einzigen Dimension gemessen wird. Eine Vermessung einer dreidimensionalen Fläche ist somit nicht oder nur durch vergleichsweise häufiges Ansetzen der Maßlehre und den daraus resultierenden Ungenauigkeiten realisierbar. Andererseits weisen die Glieder der Maßlehre keine Skaleneinheiten auf, welche eine nummerische Bestimmung der Winkelverhältnisse zwischen den einzelnen Gliedern erlauben. Dies hat zur Folge, dass der Flächenverlauf nicht durch numerische Parameter bestimmbar ist. Nach einer Verstellung der Maßlehre ist der gemessene Flächenverlauf somit auch nicht frei reproduzierbar. Aus der Druckschrift US1129081 ist eine Messvorrichtung zur Bestimmung eines Oberflächenverlaufs eines menschlichen Körpers bekannt. Es ist die Aufgabe der vorliegenden Erfindung eine Messvorrichtung der eingangs beschriebenen Art zur Verfügung zu stellen, die die Nachteile des Standes der Technik nicht aufweist und darüber hinaus einfach zu handhaben ist und vergleichsweise kostengünstig herstellbar ist und insbesondere eine zuverlässige Reproduktion der Oberfläche gestattet.

Diese Aufgabe wird erfindungsgemäß durch eine Messvorrichtung zur Bestimmung eines Oberflächenverlaufs an einem Menschen oder Tier gemäß Anspruch 1 sowie durch ein Verfahren und eine Verwendung der nebengeordneten Ansprüche gelöst. Vorteilhafte Ausgestaltungen und Weiterbildungen der vorliegenden Erfindung sind den Unteransprüchen, sowie der Beschreibung unter Bezugnahme auf Zeichnungen entnehmbar. Es ist darauf hinzuweisen, dass die in den Patentansprüchen einzeln aufgeführten Merkmale in beliebiger, technologisch sinnvoller Weise miteinander kombiniert werden können und weitere Ausgestaltungen der Erfindung aufzeigen. Die Beschreibung, insbesondere im Zusammenhang mit den Figuren, charakterisiert und spezifiziert die Erfindung zusätzlich.

Die erfindungsgemäße Messvorrichtung weist eine Hauptkette mit mehreren Hauptkettengliedern auf, die durch ein Drehgelenk miteinander verbunden sind. Die Vorrichtung zeichnet sich dadurch aus, dass eine Skalierung oder Sensoren an den Drehgelenken vorgesehen sind, mittels derer jeweils der Winkel zwischen zwei Kettengliedern ablesbar beziehungsweise bestimmbar ist.

Der Begriff Skala bzw. Skalierung im Sinne der Erfindung ist weit auszulegen und umfasst beispielsweise auch einen Nonius. Durch die erfindungsgemäße Messvorrichtung erfolgt eine Abtastung und insbesondere eine numerische Auswertung eines Oberflächenverlaufs, so dass in vorteilhafter Weise der Oberflächenverlauf trotz eines möglicherweise komplexen Verlaufs in einfacher Weise dokumentierbar, vergleichbar und/oder reproduzierbar ist. Im Allgemeinen erfolgt die Anpassung der Messvorrichtung an die Oberfläche durch einen einfachen der Oberfläche zumindest angenäherten Formschluss zwischen der Messvorrichtung und der Oberfläche, so dass die Messvorrichtung auch von einem Laien leicht zu bedienen ist. Der modulare Aufbau aus einzelnen Kettengliedern ermöglicht darüber hinaus eine vergleichsweise vielseitige und präzise Anpassung der Messvorrichtung an unterschiedlich große und unterschiedlich geformte Oberflächenverläufe. Der Aufbau aus vorzugsweise einzelnen gleichen oder zumindest ähnlichen Kettengliedern ermöglicht darüber hinaus eine vergleichsweise kostengünstige Herstellung der Messvorrichtung.

In vorteilhafter Weise wird durch das Vorsehen der Skalen eine numerische Auswertung der Relativpositionen zweier benachbarter Kettenglieder ermöglicht. Dies hat den Vorteil, dass nach dem Anpassen der Messvorrichtung an den zu messenden Oberflächenverlauf die Position eines jeden Kettengliedes der Messvorrichtung eindeutig numerisch, dokumentierbar und reproduzierbar bestimmt ist. Es ist ferner so möglich eine Fernübertragung der Messwerte vorzunehmen, beispielswiese eine Übertragung auf akustischem, insbesondere sprachlichem oder elektronischem Weg. Es reicht folglich aus, nach der Anpassung der Messvorrichtung die entsprechenden Skalenwerte der jeweiligen Skalen zu notieren, um den gesamten Oberflächenverlauf eindeutig zu dokumentieren. Auf diese Weise ist beispielsweise ein Vergleich von gemessenen Skalenwerten mit früheren Skalenwerten möglich, wodurch beispielsweise die Veränderung der Oberfläche, beispielsweise eines Pferderückens, feststellbar ist, was von orthopädischem Interesse sein kann. Ferner ist eine Reproduktion und mechanische Nachbildung des Oberflächenverlaufs an einem von dem Messort entfernten Ort möglich. So können die Skalenwerte beispielsweise schriftlich oder fernmündlich an eine Sattlerei übertragen werden, welche den Pferderückenverlauf daraufhin vor Ort reproduzieren kann und somit ein optimal an den Pferderückenverlauf angepasster Sattel von der Sattlerei herzustellen ist, ohne dass der Sattler das Pferd eigenhändig vermessen muss. Dies wird dadurch begünstigt, dass die Messvorrichtung aus den erfindungsgemäßen Kettengliedern im Gegensatz zu elektronischen Verfahren zur Bestimmung des Pferderückens vergleichsweise einfach vom jeweiligen Pferdebesitzer selbst zu bedienen ist.

Aufgrund der Eigenschaft, dass die Messvorrichtung durch die Vielzahl von Drehgelenken zusammenklappbar ist, kann die Messvorrichtung raumsparend gelagert und leicht transportiert werden und beispielsweise zwischen der Sattlerei und dem Pferdebesitzer in einfacher Weise auf dem Postweg versandt werden. Andererseits ist durch die vergleichsweise niedrigen Herstellungskosten beispielsweise eine flächendeckende Ausstattung von Pferdebetrieben mit der Messvorrichtung denkbar.

In einer alternativen Ausführungsform der vorliegenden Erfindung ist denkbar, die Drehgelenke mit Sensoren auszustatten, so dass die relative Winkelstellung, also zugehörige "Skalenwerte", elektronisch ausgelesen und bevorzugt drahtlos an eine Auswerte- und/oder Anzeigeeinheit übertragen werden.

Gemäß einer bevorzugten Weiterbildung ist vorgesehen, dass die Messvorrichtung wenigstens eine, bevorzugt mehrere, Nebenketten aufweist, mittels denen eine dreidimensionale Erfassung der Oberfläche ermöglicht ist. Beispielsweise sind die Nebenketten bei deren geradliniger Ausrichtung im Wesentlichen parallel zueinander angeordnet. So kann durch die Mehrzahl der Nebenketten die Rippenwölbung an unterschiedlichen Bereichen des Pferderückens vermessen werden. Die Nebenketten sind dabei insbesondere auf beiden Seiten der Hauptkette angeordnet, wobei die Nebenketten auf beiden Seiten vorzugsweise zueinander spiegelsymmetrisch bezüglich der Hauptkette ausgebildet sind.

Die Nebenkette ist bevorzugt an einem Kettenglied der Hauptkette befestigt, das jeweils zwei entlang einer ersten Richtung, im folgenden auch Hauptrichtung genannt, gegenüberliegende erste Endabschnitte aufweist, welche jeweils einen ersten Gelenkbeschlag umfassen und wobei wenigstens eines der Kettenglieder ferner wenigstens einen zur ersten Richtung winklig angeordneten, in eine Nebenrichtung weisenden, zweiten Endabschnitt aufweist, welcher einen zweiten Gelenkbeschlag umfasst, an dem wenigstens ein weiteres Kettenglied der Nebenkette vorgesehen ist.

In vorteilhafter Weise sind zumindest teilweise die Hauptkettenglieder und/oder Nebenkettenglieder baugleich, so dass allein durch die Verbindung einer Mehrzahl der baugleichen Kettenglieder eine Messvorrichtung zur Abbildung eines größeren Oberflächenverlaufs realisierbar ist. Eine derartige Messvorrichtung ist dabei vorteilhafterweise vielseitig anpassbar und vergleichsweise kostengünstig herstellbar. Somit ist die Messvorrichtung modular aus dem baugleichen Kettenglied herstellbar, da durch das einfache Zufügen oder Abnehmen von weiteren Kettengliedern die Größe der Messvorrichtung in gewünschter Weise einstellbar und an den zu vermessenden Oberflächenverlauf anpassbar ist.

Die Haupt- und/der Nebenkettenglieder umfassen beispielsweise Laschen, die vorzugsweise aus Pappe, Holz, Kunststoff oder Metall, insbesondere Aluminium hergestellt sind.

Gemäß einer bevorzugten Weiterbildung ist vorgesehen, dass die Hauptkette ein Kettenglied aufweist, an dem zwei oder mehr Nebenketten befestigt sind. Das Kettenglied ist somit vorzugsweise kreuzförmig ausgebildet. In vorteilhafter Weise ermöglicht eine derartige bezüglich der Hauptkette symmetrische Ausbildung der Vorrichtung die Abtastung und Abtastung eines bezüglich des Wirbelverlaufs symmetrischen Rückens, insbesondere eines Pferderückens, wobei die Hauptkette dem Verlauf der Wirbelsäule folgt und die im Wesentlichen auf beiden Seiten der Wirbelsäule rechtwinklig zur Wirbelsäule verlaufenden Rippenwölbungen des Pferderückens mittels der Nebenketten abgetastet wird.

Gemäß einer bevorzugten Weiterbildung ist vorgesehen, dass die ersten Gelenkbeschläge erste Bohrungen parallel zur zweiten Richtung und die zweiten Gelenkbeschläge zweite Bohrungen parallel zur ersten Richtung aufweisen. In vorteilhafter Weise sind somit zwei entlang der ersten oder zweiten Richtung benachbarte Kettenglieder in einfacher Weise zu verbinden, in dem in den entsprechenden ersten oder zweiten Bohrungen ein Gelenkbolzen angeordnet wird. Alternativ ist denkbar, zumindest eine der ersten oder zweiten Bohrung durch gleichwirkende erste und zweite Drehachsen auszubilden und die andere der ersten und zweiten Bohrung eines benachbarten Kettenglieds auf die erste oder zweite Drehachse aufzustecken. Anstelle der ersten oder zweiten Bohrung ist in diesem Fall auch ein Kettenglied mit ersten oder zweiten Clipsen denkbar, welche auf die erste oder zweite Drehachse aufgeclipst werden. Für einen Fachmann ist selbstverständlich, dass auch jede andere bekannte Möglichkeit einer gelenkig Verbindung zweier benachbarter Kettenglieder denkbar ist, solange die zwei benachbarten Kettenglieder dabei drehgelenkig miteinander verbunden werden. Der Gelenkbolzen umfasst beispielsweise eine Schraube, eine Klammer und/oder Niete. Bei einer alternativen Ausführung des Kettenglieds und des weiteren Kettenglieds aus Pappe ist denkbar, dass der Gelenkbolzen eine kopfklammer bzw. Musterklammer umfasst.

Gemäß einer bevorzugten Weiterbildung ist vorgesehen, dass wenigstens ein erster Gelenkbeschlag und/oder ein zweiter Gelenkbeschlag jeweils eine Skala mit Skalenteilen aufweisen, wobei die Skalenteile des ersten Gelenkbeschlags im Wesentlichen parallel zum Umfang der ersten Bohrung und die Skalenteile des zweiten Gelenkbeschlags im Wesentlichen parallel zum Umfang der zweiten Bohrung angeordnet sind. In vorteilhafter Weise ist somit der Winkel zwischen zwei benachbarten Kettengliedern an der Skala unmittelbar ablesbar. In einer bevorzugten Ausführungsform umfasst dabei eines der Kettenglieder einen Gelenkbeschlag mit einer nur einen Skalenstrich aufweisenden Skala, während der mit dem Gelenkbeschlag drehgelenkig verbundene, weitere Gelenkbeschlag des benachbarten weiteren Kettengliedes eine Skala mit einer Vielzahl von Skalenstrichen aufweist, welche mit Skalenwerten durchnummeriert sind. Der eine Skalenstrich des Kettengelenks markiert somit einen bestimmten Skalenwert auf der Skala des weiteren Kettenglieds, welcher von dem Winkel zwischen dem Kettenglied und dem weiteren Kettenglied abhängt.

In einer alternativen, besonders einfachen Ausführungsform ist denkbar, dass die Skala am Gelenkbeschlag und am mit dem Gelenkbeschlag verbundenen weiteren Gelenkbeschlag jeweils nur einen Skalenteil umfasst, welcher durch einen Markierungsstrich gebildet wird, welcher sich über beide Gelenkbeschläge erstreckt. Dieser Markierungsstrich wird vorzugsweise von dem Benutzer der Messvorrichtung nach Anpassung an die zu vermessende Oberfläche mittels eines Stifts erzeugt, wobei durch den Markierungsstrich die Winkellage zwischen dem Gelenkbeschlag und dem weiteren Gelenkbeschlag eindeutig festgelegt wird. Die Messvorrichtung ist dabei vorzugsweise aus Pappe, Holz und/oder Kunststoff gefertigt und als vergleichsweise kostengünstig herstellbare und besonders günstig zu versendende Einweg- oder Mehrwegmessvorrichtung vorgesehen. Anhand des Markierungsstrichs ist eine Rekonstruktion der vermessenden Oberfläche möglich, welche anschließend, insbesondere bei der Sattlerei, beispielsweise auf eine weitere Messvorrichtung, welche aus Metall oder Kunststoff gefertigt ist und eine Mehrzahl von nummerischen Skalenwerten umfasst, übertragen wird. Der Markierungsstrich umfasst vorzugsweise einen Teilstrich auf dem Gelenkbeschlag und einen weiteren Teilstrich auf dem weiteren Gelenkbeschlag, welche in der festgelegten Winkellage im Wesentlichen miteinander fluchten.

Gemäß einer bevorzugten Weiterbildung ist vorgesehen, dass die ersten Gelenkbeschläge entlang der zweiten Richtung und die zweiten Gelenkbeschläge entlang der ersten Richtung zueinander versetzt angeordnet sind, so dass in vorteilhafter Weise eine Kette aus einer Vielzahl von entlang der ersten oder zweiten Richtung benachbarten Kettengliedern und weiteren Kettengliedern bezüglich der ersten oder zweiten Richtung im Wesentlichen gerade ausgebildet ist.

Gemäß einer bevorzugten Weiterbildung ist vorgesehen, dass der Abstand zwischen den ersten Gelenkbeschlägen ungleich dem Abstand zwischen den zweiten Gelenkbeschlägen ist. In vorteilhafter Weise ist somit das Kettenglied an den Oberflächenverlauf des Menschen oder des Tieres universell anpassbar. Beispielsweise sind die Abstände zwischen den ersten Gelenkbeschlägen, d.h. entlang der ersten Richtung größer, als die Abstände entlang der zweiten Richtung, um entlang der ersten Richtung eine Wirbelsäule und entlang der zweiten Richtung eine Rippenwölbung eines Pferdes abzumessen. Dies ist besonders dadurch vorteilhaft, da die Krümmung der Wirbelsäule naturgemäß deutlich weniger stark, als die Krümmung der Rippenwölbung ist. Besonders bevorzugt entspricht der Abstand zwischen den ersten Gelenkbeschlägen im Wesentlichen einer mittleren Wirbellänge der Wirbelsäule eines Pferdes, um eine möglichst naturgetreue Abbildung der Wirbelsäule zu erhalten. Alternativ ist auch denkbar, dass entlang der ersten oder zweiten Richtung unterschiedliche Kettenglieder unterschiedliche Abstände zwischen den ersten oder den zweiten Drehgelenken aufweisen.

Bei einer weiteren vorteilhaften Ausgestaltung ist eine eindeutig jedes Kettenglied identifizierende Markierung, beispielsweise Nummerierung, auf jedem Kettenglied der Haupt- und/oder Nebenkette vorgesehen, um die Übertragung der Skalenwerte zu erleichtern.

In der vorliegenden Erfindung umfasst die Messvorrichtung wenigstens ein Mittel zur Bestimmung wenigstens einer Neigungsrichtung eines Kettengliedes. Anhand dieser "Bezugsausrichtung" eines oder mehrerer Kettenglieder ist die Lage der abgetasteten Oberfläche im Raum reproduzierbar definiert. Diese Information kann wichtig sein, um beispielsweise die Lage der abgetasteten Oberfläche im Raum zu analysieren. Insbesondere bei der Sattelherstellung ist diese Information wichtig, um beispielsweise die Sitzpositionierung des Reiters zu ermitteln und dann mittels dieser Information die Dimensionierung, Auslegung bzw. Anordnung der Sattelblätter, Pauschen und Steigbügelhalter festzulegen. Bevorzugt handelt es sich bei den Mitteln zur Bestimmung wenigstens einer Neigungsrichtung um eine Wasserwaagenlibelle mit Skalierung. Ein derartiges Mittel kann an einem Kettenglied der Haupt- und/oder Nebenrichtung vorgesehen sein. Bevorzugt ist die Wasserwaagenlibelle an einem Kettenglied der Hauptrichtung vorgesehen. Bevorzugt handelt es sich um eine gegenüber dem Kettenglied verschwenkbare Libelle, die so angeordnet ist, dass die Neigung des Kettenglieds betreffend dessen Längsausrichtung relativ zum Schwerefeld gemessen wird. Noch bevorzugter ist die Libelle unmittelbar an wenigstens einem benachbart zur Mitte der Hauptkette benachbarten Kettenglied befestigt.

Gemäß einer bevorzugten Weiterbildung ist vorgesehen, dass die Drehgelenke der Hauptkettenglieder und/oder Nebenkettenglieder Rastmittel, beispielsweise Formschlussmittel aufweisen. Beispielsweise weisen die ersten Gelenkbeschläge eine erste Riffelung, und/oder die zweiten Gelenkbeschläge eine zweite Riffelung, auf. In vorteilhafter Weise wird somit eine Fixierung des Winkels zwischen zwei benachbarten Kettengliedern erzielt, so dass eine versehentliche Verstellung des gemessenen Winkels unterbunden wird. Dazu greifen vorzugsweise zwei benachbarte Riffelungen ineinander, so dass beispielsweise eine Verrastung der Formschlussmittel in der jeweiligen Winkellage erzielt wird. Durch die Verrastung wird die Sicherheit bei der Bedienung der Messvorrichtung erhöht, da nur definierte Ausrichtungen ermöglicht sind und die durch die fehlerhafte Ablesung sich ergebende und gegebenenfalls sich mit der Kettenlänge "potenzierenden" Fehler vermieden werden.

Die Formschlussmittel werden dabei vorzugsweise mittels eines Spannelements aufeinandergedrückt, so dass eine Verstellung der Winkellage lediglich bei einer Überwindung der durch das Spannelement auf die Formschlussmittel erzeugten Spannkraft möglich ist.

In einer bevorzugten Ausführungsform sind dabei die erste Riffelung jeweils bezüglich der ersten Bohrung und die zweite Riffelung jeweils bezüglich der zweiten Bohrung in radialer Richtung angeordnet. Besonders bevorzugt umfasst die erste und zweite Riffelung einen Abstand der einzelnen Riffel, welche dem Abstand der Skalenteile der ersten und zweiten Skalen entspricht, so dass das Ablesen der Skalenteile deutlich erleichtert wird, da in diesem Fall eine Winkeleinstellung, welche zwischen den Skalenteilen liegt, nicht möglich ist.

Gemäß einer bevorzugten Weiterbildung ist vorgesehen, dass das Drehgelenk Reibschlussmittel aufweist. In vorteilhafter Weise wird durch das Reibmittel beim Zusammenwirken zweier benachbarter und drehbar miteinander verbundener Gelenkbeschläge der zur Relativverstellung der beiden Gelenkbeschläge benötigte Kraftaufwand deutlich erhöht, so dass eine versehentliche Winkelverstellung unterbunden wird. Das Reibschlussmittel umfasst dazu vorzugsweise eine Kontaktfläche, welche insbesondere konzentrisch um die Bohrung angeordnet ist und auf eine komplementäre weitere Kontaktfläche insbesondere mittels der Schraube, Klammer, Niete und/oder Feder gepresst wird, so dass zwischen der Kontaktfläche und der weiteren Kontaktfläche ein Reibschluss erzeugt wird. Vorzugsweise sind die Kettenglieder dabei aus Pappe gefertigt. Vorzugsweise umfasst der Gelenkbolzen eine Feder zur Erzeugung einer kraft- und/oder formschlüssigen Verbindung zwischen der ersten Riffelung und einer weiteren Riffelung des weiteren Gelenkbeschlags. In vorteilhafter Weise werden durch den von der Feder ausgehenden Federdruck die Riffelung eines Kettenglieds und die entsprechende Riffelung eines benachbarten Kettenglieds zusammengedrückt, so dass der Winkel zwischen dem Kettenglied und dem benachbarten Kettenglied nur bei Überwindung der Federkraft einstellbar ist. Das Kettenglied und das benachbarte weitere Kettenglied sind relativ zueinander durch das gegenseitige Einrasten der Riffelungen fixiert. Eine versehentliche Verstellung des Winkels wird somit unterdrückt. Es ist denkbar, dass die Reibung magnetisch oder elektromagnetisch bewirkt wird.

Gemäß einer bevorzugten Ausgestaltung sind Verriegelungsmittel vorgesehen, die die Drehgelenke der erfindungsgemäßen Messvorrichtung in der jeweiligen Winkellage fixieren können und beispielsweise die Formschlussmittel in ihrer formschlüssigen Stellung verriegeln. Beispielsweise ist wenigstens ein verschiebbarer Riegel vorgesehen, der die Formschlussmittel in ihrer eingerasteten Stellung verriegelt. Beispielsweise wird der Riegel von einem verdrehbaren Nocken in die Verriegelungsstellung angetrieben, wobei dessen Rückstellung in die Entriegelungsstellung mittels elastischer Vorspannung durch eine Feder erreicht wird.

Die "Versteifbarkeit" der Messvorrichtung durch die Reibschlussmittel oder die Formschlussmittel, noch bevorzugter in Kombination mit Verriegelungsmitteln hat den Vorteil, dass die Messvorrichtung nicht nur maßnehmend verwendet werden kann sondern darüber hinaus maßprüfend verwendet werden kann, indem beispielsweise ein Oberflächenverlauf anhand einer voreingestellten Messvorrichtung im Hinblick auf Abweichungen überprüft wird. Beispielsweise wird so ein hergestellter Sattel mittels einer Messvorrichtung überprüft, die auf die zuvor am Pferd genommenen Skalenwerte eingestellt und durch die Reib-, Formschlussmittel und/oder Verriegelungsmittel "verseift" wurde.

Gemäß einer weiteren, bevorzugten Ausführungsform ist vorgesehen, dass das Hauptkettenglied, an dem die Nebenkette befestigt ist, eine Schwenkverstellung der Nebenkette gegenüber der Hauptkette ermöglicht. In einer besonders bevorzugten Ausführungsform umfasst die Schwenkverstellung weitere Form- und/oder Reibschlussmittel zur Verrastung und/oder Fixierung der jeweiligen Schwenkstellung und vorzugsweise eine Skala, wodurch die jeweilige Schwenkstellung numerisch festgelegt und reproduzierbar ist.

Gemäß einer bevorzugten Weiterbildung ist vorgesehen, dass der Abstand zwischen zwei weiteren Endabschnitten wenigstens eines Kettengliedes mittels einer Längsverstellung einstellbar ist, wobei die Längsverstellung vorzugsweise ein Langloch und eine entlang des Langlochs angeordnete weitere Skala aufweist. Vorzugsweise weist die Längsverstellung ebenfalls Form- und/ oder Reibschlussmittel, insbesondere eine Riffelung und/oder Kontaktfläche auf, so dass eine unbeabsichtigte Längsverstellung verhindert wird.

Ein weiterer Gegenstand der vorliegenden Erfindung ist ein Verfahren zur Bestimmung eines Oberflächenverlaufs eines menschlichen oder tierischen Körpers, bevorzugt eines Pferderückens, mit einer erfindungsgemäßen Messvorrichtung, wobei im ersten Verfahrensschritt die Messvorrichtung auf der Oberfläche angeordnet wird und die Hauptglieder und gegebenenfalls die Nebenglieder an den Oberflächenverlauf angepasst werden und wobei in einem zweiten Verfahrensschritt Skalenwerte der Messvorrichtung abgelesen werden.

In einer bevorzugten Ausgestaltung des Verfahrens wird im zweiten Verfahrensschritt wenigstens eine Neigungsrichtung eines Haupt- oder Nebenkettengliedes, bevorzugt eines Hauptkettengliedes, bestimmt. In vorteilhafter Weise wird der Oberflächenverlauf, also der Pferderücken, somit eindeutig numerisch ermittelt und ist somit jederzeit frei reproduzierbar. Insbesondere ist ein Vergleich der gemessenen Skalenwerte mit früheren Skalenwerten möglich.

Ein weiterer Gegenstand der vorliegenden Erfindung ist eine Verwendung der erfindungsgemäßen Messvorrichtung zur Herstellung, Anpassung und/oder Kontrolle einer Pferdeausrüstung, insbesondere eines Sattels. In vorteilhafter Weise wird somit der Pferderücken in einer vergleichsweise einfach handhabbaren Weise vollständig vermessen, wobei der Oberflächenverlauf des Pferderückens numerisch in Form der Skalenwerte dokumentierbar wird. Dies ermöglicht eine Fertigung und/oder Anpassung der Pferdeausrüstung allein auf der Grundlage der Skalenwerte. Die Skalenwerte werden dabei vorzugsweise als Eingangsparameter für eine CNC-Maschine genutzt, so dass die Pferdeausrüstung maschinell herstellbar ist und gleichzeitig individuell an das entsprechende Pferd angepasst ist. Dies ermöglicht insbesondere eine, beispielsweise elektronische und/oder fernmündliche Bestellung von individualisierter Pferdeausrüstung, wobei der Pferdebesitzer die entsprechenden Herstellungsparameter in einfacher Weise selbst ermittelt, so dass erhebliche Kosteneinsparungen zu erzielen sind. Bei dem erfindungsgemäß vorgesehenen Kontrollschritt ist eine Überprüfung von Pferdeausrüstung mittels der Messvorrichtung vorgesehen, wobei beispielsweise in dem dritten Verfahrensschritt die Messvorrichtung oder eine gemäß den Skalenwerten neu eingestellte Messvorrichtung in eine bereits vorhandene Pferdeausrüstung, bspw. in einen Sattel, eingelegt wird, so dass eine Abweichung zwischen der Pferdeausrüstung und dem Oberflächenverlauf des Pferderückens anhand einer Abweichung zwischen der Pferdeausrüstung und der Messvorrichtung zu analysieren ist.

Beispielsweise wird die Messvorrichtung zur individuellen Anpassung der Sattelaufliegefläche eines Sattelbocks an einen durch die Messvorrichtung vorher ermittelten Pferderücken verwendet. Ein Sattelbock dient dem Testen eines individuell hergestellten Sattels. Beispielsweise wird der Sattelbock entsprechend den durch die Messvorrichtung ermittelten Skalenwerten produziert oder weist eine Verstellmöglichkeit zur Anpassung der Sattelauflagefläche.

Ausführungsbeispiele der Erfindung sind in den Zeichnungen dargestellt und in der nachfolgenden Beschreibung näher erläutert. Es zeigen
Figur 1 eine schematische Perspektivansicht zweier Kettenglieder, nämlich ein Hauptkettenglied 44 gemäß einer ersten Ausführungsform und ein Nebenkettenglied 54 gemäß einer ersten Ausführungsform;
Figur 2 eine Detailansicht des Drehgelenks zwischen dem Hauptkettenglied 44 und dem Nebenkettenglied aus Figur 1;
Figur 3a eine schematische Perspektivansicht zweier Kettenglieder, nämlich dem Hauptkettenglied 44 gemäß der ersten Ausführungsform und ein Nebenkettenglied 54' gemäß einer zweiten Ausführungsform;
Figur 3b eine schematische Aufsicht zweier Kettenglieder, nämlich dem Hauptkettenglied 44' gemäß einer zweiten Ausführungsform und ein Nebenkettenglied 54 gemäß der ersten Ausführungsform;
Figur 3c eine schematische Perspektivansicht zweier Kettenglieder, nämlich dem Hauptkettenglied 44 gemäß der ersten Ausführungsform und ein Nebenkettenglied 54" gemäß einer dritten Ausführungsform;
Figur 4 eine schematisch Ansicht einer maßnehmende Verwendung der erfindungsgemäßen Messvorrichtung an einem Pferderücken;
Figur 5 eine Detailansicht der Figur 4;
Figur 6 eine Detailansicht der Messvorrichtung aus Figur 4 bei Betrachtung in Richtung des Wirbelsäulenverlaufs;
Figuren 7a, 7b schematische Ansichten einer Messvorrichtung gemäß weiterer Ausführungsform der vorliegenden Erfindung;
Figuren 8a, 8b schematische Ansichten einer Messvorrichtung gemäß noch einer weiteren Ausführungsform der Erfindung;
Figur 9 eine Detailansicht der Mittel 200 zur Bestimmung einer Neigungsrichtung eines Hauptkettengliedes
Figuren 10 bis 12 eine weitere Ausführungsform der Messvorrichtung bei der die Haupt- und/oder Nebenkettenglieder ferner Verriegelungsmittel aufweisen.

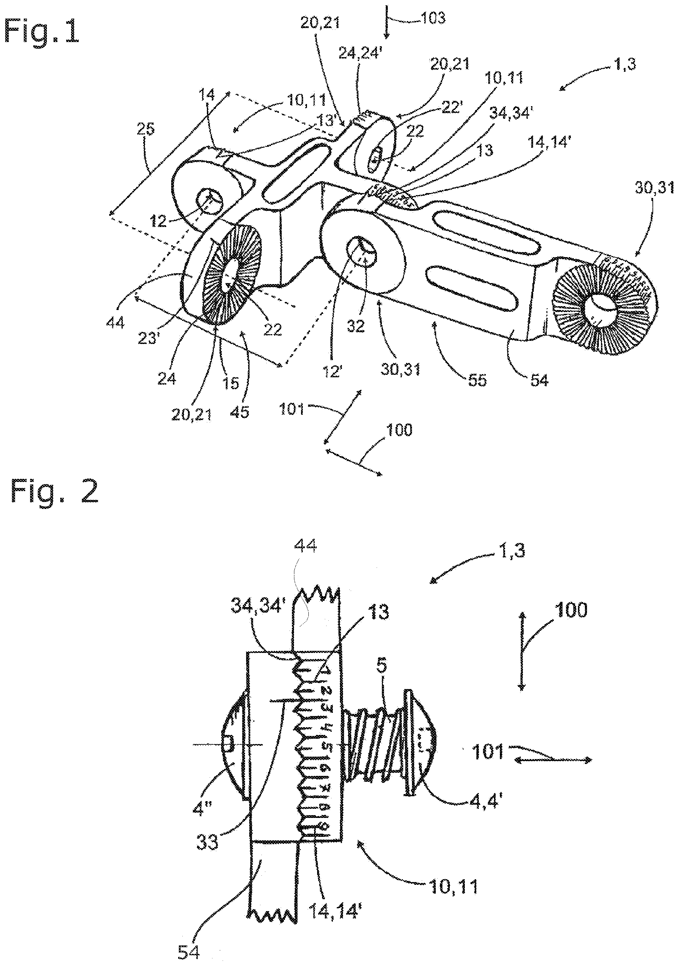

In Figur 1 ist eine schematische Perspektivansicht eines Teils der erfindungsgemäßen Messvorrichtung 1 gemäß einer ersten Ausführungsform dargestellt, wobei das Kettenglied 44 der Hauptkette insgesamt vier Gelenkbeschläge 11,21 aufweist, welche alle in einer Haupterstreckungsebene 102 des Kettenglieds 44 liegen. Zwei erste Gelenkbeschläge 11 sind dabei an zwei gegenüberliegenden ersten Endabschnitten 10 des Kettenglieds 44 angeordnet, während zwei zweite Gelenkbeschläge 12 an zwei gegenüberliegenden zweiten Endabschnitten 20 angeordnet sind. Die ersten Gelenkbeschläge 11 sind im Wesentlichen entlang einer ersten Richtung 100 und die zweiten Gelenkbeschläge 21 im Wesentlichen entlang einer zur ersten Richtung 100 senkrechten zweiten Richtung 101 angeordnet. Das Kettenglied 44 umfasst somit eine Art Kreuzstruktur und ist daher sowohl entlang der ersten Richtung 100 an beiden ersten Endabschnitten 10, als auch entlang der zweiten Richtung 101 an beiden Endabschnitten 20 mit benachbarten weiteren Hauptkettengliedern (nicht dargestellt) beziehungsweise Nebenkettengliedern 54 drehgelenkig zu verbinden. Die ersten Gelenkbeschläge 11 weisen dazu jeweils eine erste Bohrung 12 und als erstes Formschlussmittel 14 in Form 10 einer ersten Riffelung 14' und die zweiten Gelenkbeschläge 21 jeweils eine zweite Bohrung 22 und ein zweites Formschlussmittel 24 in Form einer zweiten Riffelung 24' auf. Ferner weist jeweils eines der ersten Gelenkbeschläge 11 eine Skala 13 mit einer Mehrzahl von entlang des Umfangs der entsprechenden Bohrung 12 angeordneten Skalenteilen auf, wobei das andere der beiden ersten 15 Gelenkbeschlägen 11 eine Skala 13 mit genau einem Skalenstrich 13' aufweist. Die zwei zweiten Gelenkbeschläge 21 sind analog dazu mit zwei zweiten Skalen 23 ausgestattet. Aus Gründen der Übersichtlichkeit ist beispielhaft lediglich ein benachbartes Nebenkettenglied 54 dargestellt, welches durch ein Zusammenwirken eines ersten Gelenkbeschlags 11 mit einem weiteren Gelenkbeschlag 31 dieses Kettenglieds 54 mit dem Hauptkettenglied 44 drehgelenkig verbunden ist. Die erste Bohrung 12 ist dabei deckungsgleich zu einer weiteren Bohrung 32 des weiteren Gelenkbeschlags 32 angeordnet, wobei beide Bohrungen 12, 32 parallel zur zweiten Richtung 101 ausgerichtet sind und wobei der erste und der weitere Gelenkbeschlag 11, 31 durch einen nicht dargestellten Gelenkbolzen 3, welcher in der ersten und der weiteren Bohrung 12, 32 angeordnet ist, derart zusammengehalten werden, dass die erste Riffelung 14' in eine weitere Riffelung 34' eines komplementären, weiteren Formschlussmittel 34 des weiteren Gelenkbeschlags 31 eingreift. Der weitere Gelenkbeschlag 31 umfasst ferner eine weitere Skala 33 mit einem Skalenstrich 33', welcher zu der Mehrzahl von 30 Skalenteilen der ersten Skala 13 benachbart angeordnet ist, so dass der Skalenstrich 33' der weiteren Skala 33 in Abhängigkeit des Winkels zwischen dem Hauptkettenglied 44 und dem Nebenkettenglied 54 bezüglich einer Drehung um den Gelenkbolzen 3 einen Skalenteil des ersten Skala 13 markiert. Der zugehörige Skalenwert ist somit ein eindeutiges und reproduzierbares Maß für die Relativposition zwischen dem Hauptkettenglied 44 und dem Nebenkettenglied 54. Die erste und weitere Riffelung 14', 34' bewirken eine Verrastung zwischen dem Hauptkettenglied 44 und dem Nebenkettenglied 54 in der jeweiligen Winkelposition. Die Abstände zwischen den einzelnen Rastpositionen entsprechen dabei im Wesentlichen den Abständen zwischen den 5 einzelnen Skalenteilen, so dass immer genau ein Skalenwert der Skala 13 einer Rastposition entspricht. Es wird darauf hingewiesen, dass die drehgelenkige Verbindung der Kettenglieder untereinander kompatibel ist. Bevorzugt sind in der Richtung 101 mehrere Hauptkettenglieder 44 in der in Figur 1 gezeigten Ausrichtung drehgelenkig verbunden, um eine Hauptkette zu bilden und mehrere Nebenkettenglieder 54 in der Richtung 100 verbunden, um die Nebenkette der Messvorrichtung zu bilden.

In Figur 2 ist eine Seitenansicht aus der gemäß des in Figur 1 dargestellten Pfeils 103 dargestellt, wobei zusätzlich in Figur 2 der Gelenkbolzen 4 abgebildet ist. Der Gelenkbolzen 4 umfasst eine Schraube 4' und eine ein Innengewinde aufweisende Hülse 4", in welche die Schraube 4' eingedreht ist, so dass der erste Gelenkbeschlag 11 und der weitere Gelenkbeschlag 31 durch die Köpfe der Schraube 4' und der Hülse 4" drehgelenkig zusammengehalten werden. Ferner umfasst der Gelenkbolzen 4 eine Feder 5, welche ein Ineinandergreifen der ersten Riffelung 14' und der weiteren Riffelung 34' bewirkt, so dass zum Verstellen des Winkels zwischen dem Kettenglied 2 und dem weiteren Kettenglied 2' die Federkraft der Feder 5 überwunden werden muss, damit die erste und weitere Riffelung 14,34 außer Eingriff gebracht werden können.

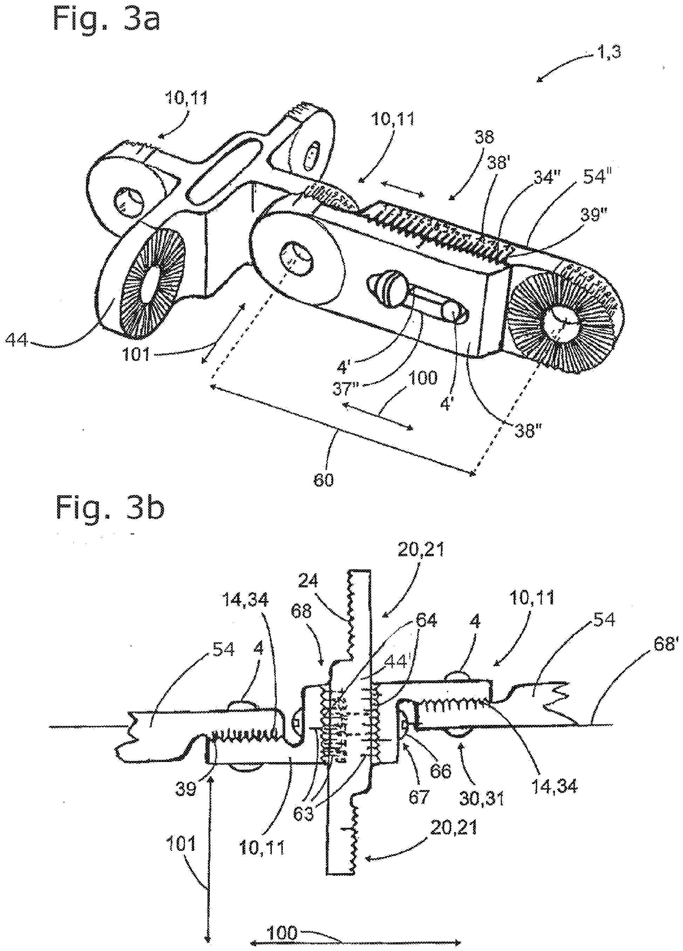

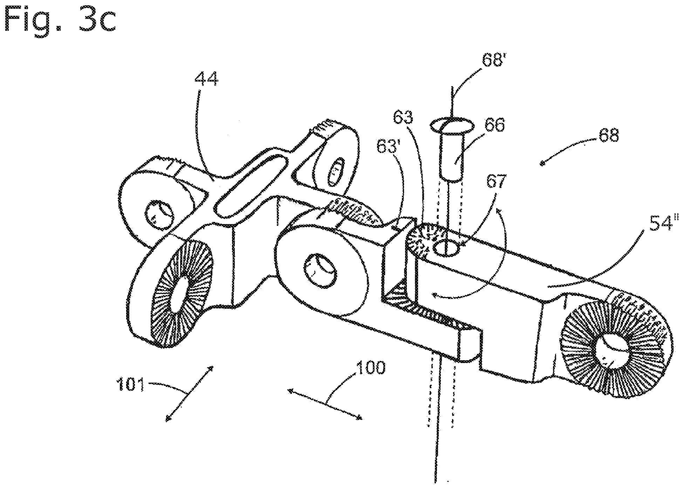

In Figur 3a ist eine schematische Perspektivansicht eines Kettenglieds 2 gemäß der ersten Ausführungsform der vorliegenden Erfindung und einer Anordnung 3 eines Kettenglieds gemäß einer zweiten Ausführungsform der vorliegenden Erfindung dargestellt, wobei die Figur 3 im Wesentlichen der Figur 1 gleicht, wobei das Nebenkettenglied 54' mittig entlang der ersten Richtung 100 eine Längsverstellung 38 aufweist. Dazu umfasst das Kettenglied 54' ein erstes Teilstück 38' mit ersten Bohrungen 37' und ein zweites Teilstück 38" mit einem Langloch 37". Das erste und das zweiten Teilstück 38' und 38" sind mittels zweier Bolzen 4' miteinander verbunden, welche fest in den Bohrungen 37' angeordnet und längsverschieblich in dem Langloch 37" gelagert sind. Durch die Längsverschieblichkeit der Bolzen 4' ist die Länge 60 des Nebenkettenglieds 54' entlang der ersten Richtung 100 einstellbar. An dem zweitem Teilstück 38" ist ferner eine erste Skala 39" mit einem einzigen Skalenstrich und auf dem ersten Teilstück 38' eine zweite Skala 39" mit einer Mehrzahl von Skalenteilen angeordnet, so dass die Relativposition zwischen dem ersten Teilstück 38' und dem zweiten Teilstück 38" numerisch festlegbar ist. Ferner umfassen das erste und das zweite Teilstück 38', 38" Riffelungen 34", welche eine versehentliche Längsverschiebung verhindern und mittels auf den Bolzen 4' angeordneten Federn in gegenseitigem Eingriff gehalten werden.

Figur 3b ist eine Messvorrichtung mit einem Hauptkettenglied 44' gemäß einer zweiten Ausführungsform der vorliegenden Erfindung dargestellt, wobei das Kettenglied 44' zusätzlich zwei Schwenkverstellungen 68 für die Nebenketten aus den Nebenkettengliedern 54 aufweist, wobei die ersten Endabschnitte 10 gegenüber dem Kettenglied 2 mittels der Schwenkverstellungen 68 um eine Schwenkachse 68' parallel zur ersten Richtung 100 insbesondere unabhängig voneinander schwenkbar sind. Dazu weist das Kettenglied 44' eine zentrale Bohrung 67 parallel zur ersten Richtung 100 auf, in welcher ein Gelenkbolzen 66 verläuft, an welchem die ersten Endabschnitte 10 mit entsprechenden Bohrungen 67 befestigt sind. Ferner weisen die Endabschnitte 10 Formschlussmittel 64 und das restliche Kettenglied 2 entsprechend komplementäre Formschlussmittel 64' in Form von Riffelungen auf, so dass die Endabschnitte 10 in ihren jeweiligen Schwenklagen gegenüber dem restlichen Kettenglied verrastbar sind. Zur nummerischen Angabe der jeweiligen Schwenklage umfassen die Schwenkverstellungen 68 jeweils Skalen 63 mit entsprechenden Skalenteilen. Alternativ wäre es ebenso denkbar, zusätzlich oder ausschließlich die zweiten Endabschnitte 20 jeweils mittels einer Schwenkverstellung 68 an die Nebenkettenglieder 54 anzubinden.

In Figur 3c ist eine weitere Ausführungsform der Messvorrichtung gezeigt, bei der die Nebenkette mittels eines Nebenkettenglied 54" an die Hauptkette angebunden ist, das anstelle der Längsverstellung 38 eine Schwenkverstellung 68 aufweist. Das erste Teilstück 38' und das zweite Teilstück 38" umfassen dazu jeweils eine deckungsgleiche Bohrung 67 zur Aufnahme eines die Schwenkachse 68' definierenden Schwenkbolzens 66, wobei das erste und das zweite Teilstück 38', 38" um die Schwenkachse 68' gegeneinander verschwenkbar sind. Die Schwenkachse 68' verläuft dabei im Wesentlichen senkrecht sowohl zur ersten, als auch zur zweiten Richtung 100, 101. Die jeweilige Winkellage zwischen dem ersten und dem zweiten Teilstück 38',38" ist dabei mittels eines Formschlussmittels 64, insbesondere in Form von Riffelungen 64', verrastbar, so dass ein versehentliches Verschwenken unterbunden wird. Ferner weist das erste Teilstück 38' eine Skala 63 mit einer Mehrzahl von nummerierten Skalenteilen auf, 10 während das zweite Teilstück 33" eine Skala 63' mit einem einzigen Skalenstrich zur Markierung eines entsprechenden Skalenteils in Abhängigkeit der jeweiligen Winkellage zwischen dem ersten und dem zweiten Teilstück 38',38" bezüglich der Schwenkachse 68'. Alternativ ist auch denkbar, dass das benachbarte kreuzartige Hauptkettenglied 44 mit einer derartige Schwenkverstellung 68 zum Verschwenken 15 wenigstens eines der ersten und/oder zweiten Endabschnitte 10, 20 ausgestattet ist.

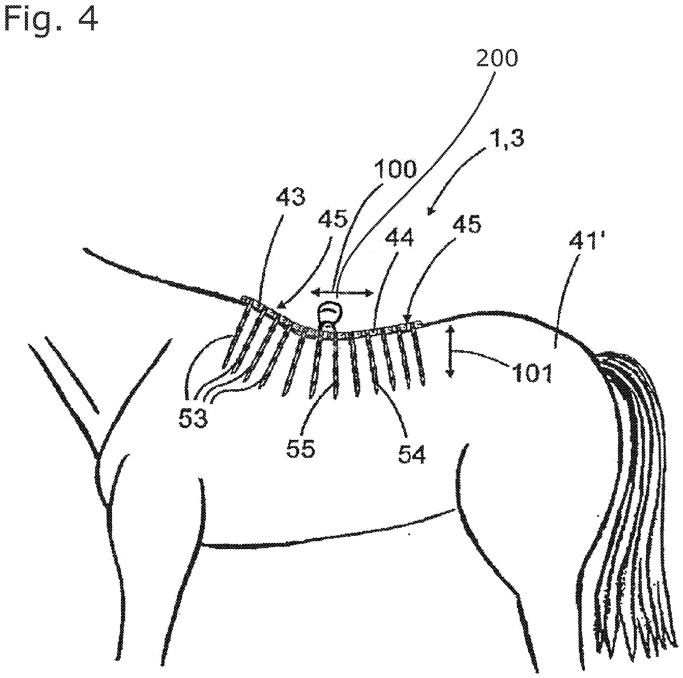

In den **Figuren 4****,** **5 und 6** sind schematische Ansichten einer Messvorrichtung 1 gemäß einer Ausführungsform der vorliegenden Erfindung dargestellt, wobei die Messvorrichtung 1 in den Figuren 4,5 und 6 beispielhaft auf einem Pferderücken 41' angeordnet ist. Die Messvorrichtung 1 umfasst dabei eine Hauptkette 43, welche sich im Wesentlichen entlang einer ersten Richtung 100 erstreckt und aus einer Vielzahl von Hauptkettengliedern 44 besteht. Die Hauptkettenglieder 44 umfassen dabei insbesondere eine Mehrzahl des in Figuren 1, 2 und 3 dargestellten erfindungsgemäßen Kettenglieds 2, welche jeweils mittels Gelenkbolzen 4 drehgelenkig miteinander verbunden sind. Ferner umfasst die Messvorrichtung 1 eine Mehrzahl von Nebenketten 53, wobei sich jede Nebenkette im Wesentlichen entlang einer zur ersten Richtung 100 senkrechten zweiten Richtung 101 erstreckt und wobei jede Nebenkette 53 aus einer Vielzahl von Nebenkettengliedern 54 besteht. Die Nebenkettenglieder 54 umfassen insbesondere jeweils eine Mehrzahl von in Figur 1 dargestellten weiteren Kettengliedern 2', welche teilweise mittels Gelenkbolzens 4 drehgelenkig miteinander verbunden sind. Die Hauptkette 43 ist im Wesentlichen entlang einer Wirbelsäule des Pferderückens 41' angeordnet, so dass durch die Relativpositionen der benachbarten Hauptkettenglieder 44 zueinander der Verlauf der Wirbelsäule nachgebildet wird, während die entlang der Hauptkette 43 21 nebeneinander angeordneten Nebenketten 53 die jeweiligen Rippenwölbungen des Pferderückens 41' entlang der Wirbelsäule nachbilden. Wie bei der Anordnung von Kettenglieder gemäß der Figuren 1 und 2 umfasst jedes der Drehgelenke 45, 55 zwischen benachbarten Hauptkettengliedern 44, zwischen benachbarten 5 Nebenkettengliedern 54 und zwischen benachbarten Haupt- und Nebenkettengliedern 44, 54 eine Verrastung mittels entsprechender Formschlussmittel 14, 24, 34 und eine Skala 13, 33, welche den Winkel zwischen den jeweiligen benachbarten Elementen nummerisch angibt. Nach einem Andrücken bzw. einer formschlüssigen Anpassung der Messvorrichtung 1 an den Pferderücken 41' in einem ersten Schritt, sind in einem zweiten Schritt die Skalenwerte an den Skalen 13, 33 der verschiedenen Drehgelenke 45, 55 abzulesen. Allein durch Angabe dieser Skalenwerte und einer Zuordnung dieser Skalenwerte zu den entsprechenden Drehgelenken 45, 55 ist die dreidimensionale Oberfläche des Pferderückens 41' eindeutig und reproduzierbar charakterisiert. Es ist ferner eine Wasserwaagenlibelleneinrichtung 200 zur Bestimmung einer Neigungsrichtung eines mittleren Hauptkettenkettengliedes 45 vorgesehen. Da die Lage des Hauptkettengliedes 45 durch den Verlauf der Wirbelsäule des Pferderückens definiert ist, reicht im vorliegenden Fall die Bestimmung einer Neigungsrichtung des Hauptkettengliedes 45 aus, um die Lage der gemessenen Oberfläche im Raum zu definieren. Entsprechend dieser zusätzlich gewonnenen Information kann der Sattel insbesondere im Hinblick auf die anvisierte Sitzposition des Reiters ausgelegt werden. Die Herstellung und/oder Anpassung einer Pferdeausrüstung, wie beispielsweise eines Reitsattels, ist somit allein auf der Basis der Skalenwerte möglich.

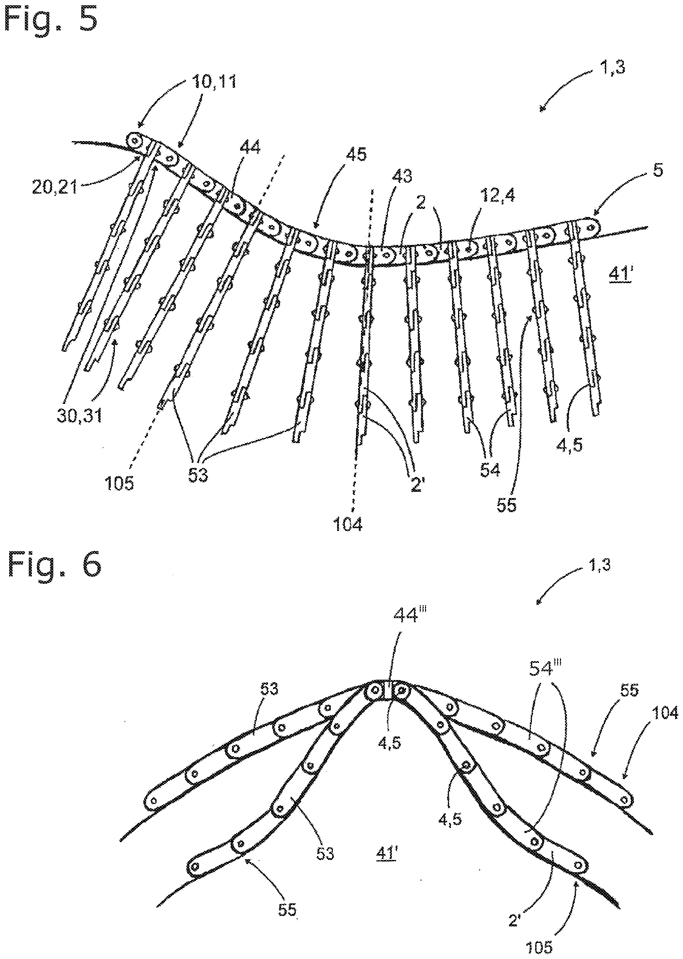

In Figur 6 ist eine Schnittdarstellung gemäß der in Figur 5 illustrierten Schnittlinien 104 und 105 schematisch dargestellt, wobei anhand der Schnittdarstellung die Nachbildung unterschiedlicher Rippenwölbungen im Widerristbereich 104' und im Rippenbereich 20 105' des Pferderückens 41' mittels unterschiedlicher Nebenketten 53 illustriert wird. In einer bevorzugten Ausführungsform sind einzelne Kettenglieder 44, 54 mit einer Schwenkverstellung 68 ausgestattet sind; so dass eine Berührung zweier benachbarter Nebenketten 53, beispielsweise bei einer entsprechend stark gekrümmten Wirbelsäule, durch eine entsprechende Verschwenkung der Nebenketten 53 unterbunden werden kann. In einer weiteren bevorzugten Ausführungsform ist denkbar die Anbindung jeder Nebenkette 53 an ein Hauptkettenglied 44 jeweils mit einer Schwenkverstellung 38 auszustatten, so dass alle Nebenketten 53 parallel zum Schwerefeld auszurichten sind. Eine derartige Ausrichtung der Nebenketten 53 könnte vorzugsweise mittels Libellen erzielt werden, 30 welche an den einzelnen Nebenkettengliedern 54 angeordnet sind.

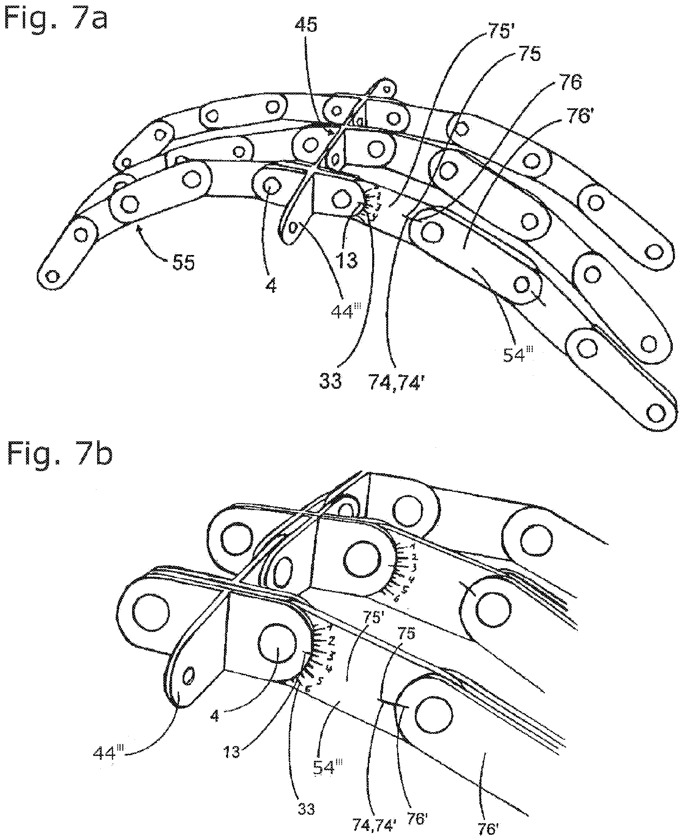

In den Figuren 7a und 7b sind schematische Perspektivansichten einer Messvorrichtung 1 gemäß einer weiteren Ausführungsform der vorliegenden Erfindung dargestellt, wobei die zweite Ausführungsform im Wesentlichen der in den Figuren 4, 5 und 6 illustrierten Messvorichtung 1 gemäß der ersten Ausführungsform gleicht aber die Hauptkettenglieder 44"' und die Nebenkettenglieder 54"' im Wesentlichen und insbesondere deren Laschen aus Pappe 5 gefertigt sind. Benachbarte Kettenglieder 44"' und 54"' sind mittels Gelenkbolzen 4 in Form von Nieten miteinander verbunden. Zur Erhöhung der Verwindungssteifigkeit sind die Kettenglieder 2 und die weiteren Kettenglieder 2' zumindest teilweise doppelwandig ausgeführt. Ferner umfassen die Drehgelenke 45, 55 im Unterschied zur zuvor gezeigten Ausführungsform bei dieser Ausführungsform keine Formschlussmittel sondern stattdessen Reibschlussmittel 71, 72, 73, wobei Kontaktflächen von benachbarten Reibschlussmitteln 71, 73 durch die Nieten aufeinander gepresst bzw. geklemmt werden, so dass zur Winkelverstellung der benachbarten Haupt- und/oder Nebenkettenglieder 44"', 54"' der Reibschluss zwischen den entsprechenden Kontaktflächen überwunden werden muss und somit die Gefahr einer versehentliehen Verstellung der Winkellage verringert wird. Die Haupt- und Nebenkettenglieder 44"', 54"' umfassen teilweise Skalen 13, 33 gemäß der ersten Ausführungsform, wobei der Winkel zwischen den entsprechenden benachbarten Haupt- und Nebenkettengliedern 44"', 54"' nummerisch ablesbar ist. Alternativ umfassen einige Haupt- und Nebenkettenglieder 44"', 54"' eine Skala 74', welche lediglich einen Markierungsstrich 74 umfasst, welcher erst durch den Benutzer der Messvorrichtung 1 beispielsweise mittels eines Filzstifts auf der Messvorrichtung 1 erzeugt wird. Dieser Markierungsstrich 74 wird in der eingestellten bzw. gemessenen Winkellage über zwei benachbarte und miteinander gelenkig verbundene Drehgelenke 75', 76' gezogen, so dass ein erster Teilstrich 75 auf dem einen Drehgelenk 75' und ein zweiter Teilstrich 76 auf dem benachbarten anderen Drehgelenk 76' entsteht und die entsprechenden Winkellage zwischen den benachbarten Drehgelenken 75', 76' durch ein Fluchten der beiden Teilstriche 75, 76 reproduzierbar festgelegt ist.

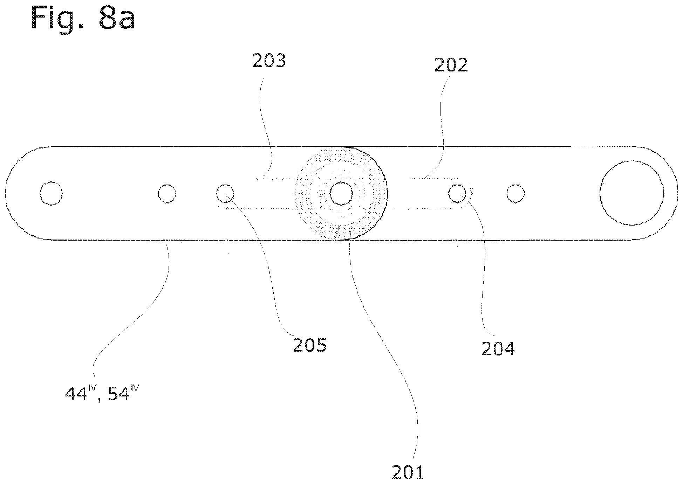

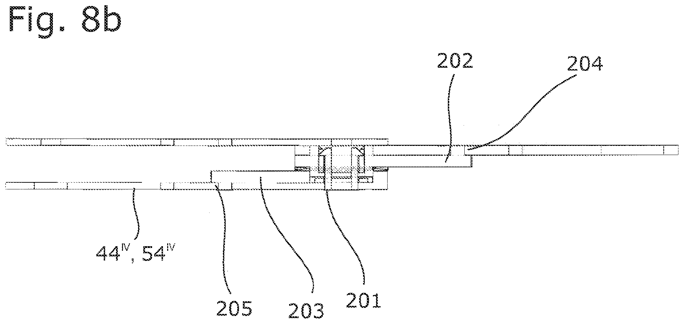

In den Figuren 8a und 8b ist eine weitere Ausführungsform der Messvorrichtung mit im Wesentlichen aus Pappe hergestellten Kettengliedern 44^{IV}, 54^{IV} der Haupt- und Nebenketten dargestellt, wobei zusätzliche Formschlussmittel 202, 203 in Form von Riffelscheiben aus Kunststoff in der Drehgelenksverbindung der Kettenglieder 44^{IV}, 54^{IV}, vorgesehen sind, deren Riffelflächen durch Federvorspannung aufeinander gedrückt werden. Diese Formschlussmittel 202, 203 sind zur Verdrehsicherung mit einem seitlichen Ausleger versehen, der jeweils mit einem Vorsprung in einen Durchbruch 204, 205 der Kettenglieder 44^{IV}, 54^{IV} eingreift.

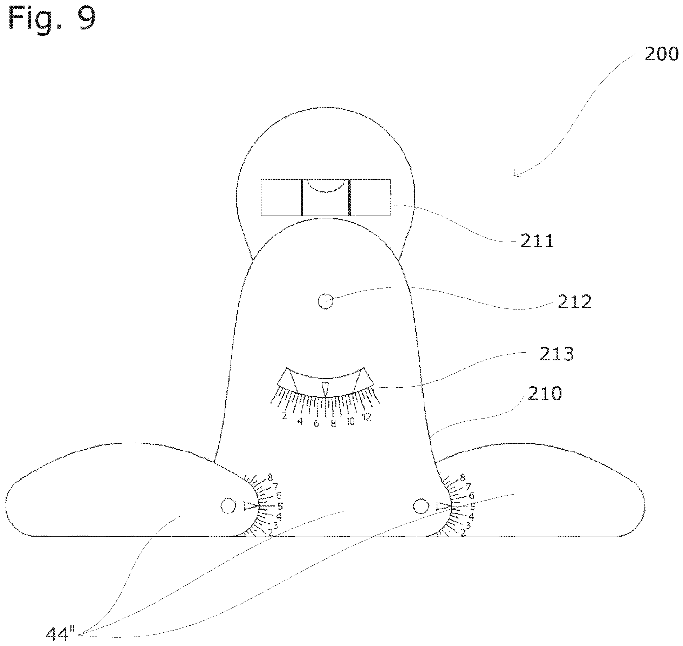

In Fig. 9 ist im Detail das Mittel 200 zur Bestimmung einer Neigungsrichtung eines Hauptkettengliedes 44" gezeigt, wobei letztere sich in ihrer Seitenkontur von den zuvor gezeigten Ausführungsformen unterscheiden. Das Mittel 200 zur Bestimmung der Neigungsrichtung umfasst eine Wasserwaagenlibelle, die verschwenkbar um eine Drehachse 212 an der Lasche 210 des Kettenglieds 44" befestigt ist. Das Maß der Verschwenkung ist an einer Skala 213 ablesbar. Es wird im Allgemeinen so vorgegangen, dass nach dem Auflegen und Anpassen der Messvorrichtung an die zu vermessende Oberfläche, der Teil mit der Wasserwaagenlibelle durch Verschwenken austariert wird und der zugehörige Skalenwert an der Skalierung 213 abgelesen wird. Sollte eine weniger genaue Ablesegenauigkeit erforderlich sein, könnte auch die Wasserwaagenlibelle starr mit dem Kettenglied verbunden sein und die Skalierung an der Libelle selbst vorgesehen sein. Es kann auch eine Libelle vorgesehen, mit der eine Austarierung in alle Richtung möglich ist und somit die Neigung des Kettenglieds bezüglich wenigstens zweier orthogonaler Richtungen ermittelbar ist.

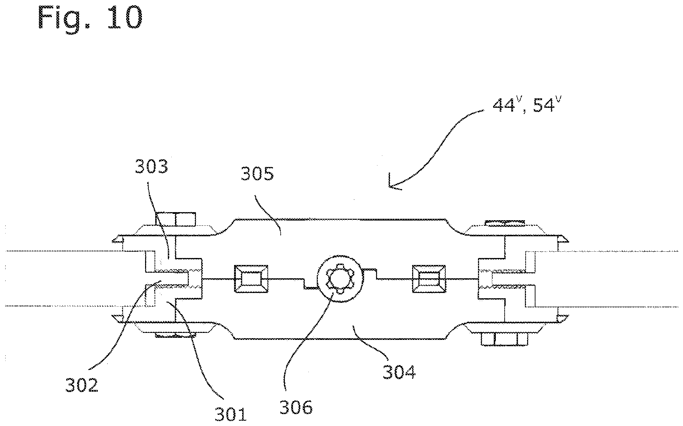

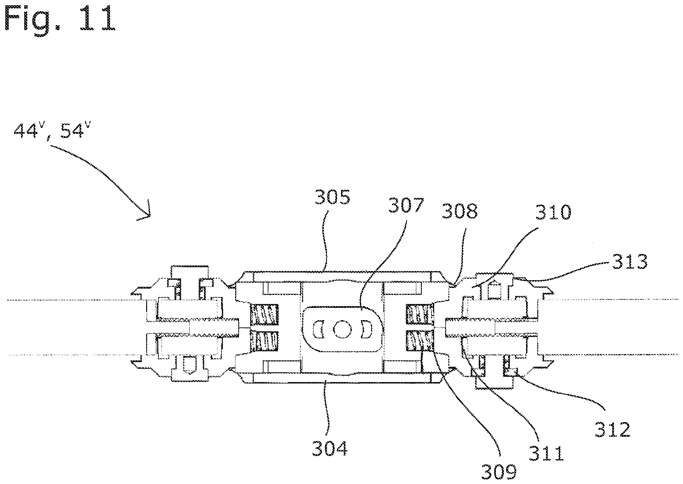

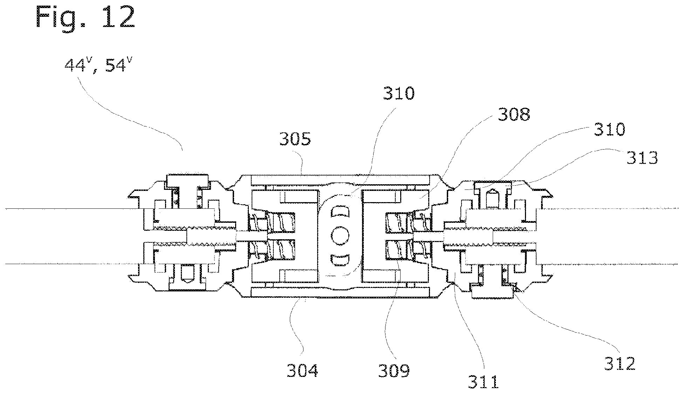

In den Figuren 10, 11, 12 ist eine weitere Ausführungsform eines Kettenglieds 44^{V}, 54^{V}, der Haupt- und/oder Nebenkette der erfindungsgemäßen Messvorrichtung gezeigt, bei dem zusätzlich Verriegelungsmittel 306, 307, 308, 309 vorgesehen sind. Die Verriegelungsmittel sind so ausgelegt, dass sie in der zugehörigen Verriegelungsstellung die ferner vorgesehenen Formschlussmittel bzw. Rastmittel 310, 311 in deren Eingriffsstellung verriegeln. Die Gelenkbeschläge dieser Ausführungsform weisen Rastmittel 310, 311 auf, die durch eine Schraubverbindung 313 und eine Federvospannung 312 aufeinandergedrückt werden und die zugehörige Riffelflächen in Eingriff gelangen, wie es in Fig. 11 gezeigt ist. Die Verriegelung der Rastmittel 310, 311 wird durch einen Nockenantrieb 306, 307 bewirkt. Dieser ist in zwei, das Kettenglied 44^{V}, 54^{V}, im Wesentlichen definierenden Halbschalen 304, 305 integriert. Durch Verstellen der von außen zugänglichen Drehachse 306, die mit einer Ansatzmöglichkeit für ein Werkzeug versehen ist, wird im Innern ein Nocken 307 verdreht, der jeweils mit seiner Nockenfläche eine Linearverschiebung eines U-förmigen Riegels 308 gegen eine Federvorspannung 309 bewirkt. Der Riegel 308 läuft gegen einen rampenförmig ausgebildeten Abschnitt der Rastmittel 310, 311 und zwingt deren Riffelflächen in Eingriff und umgreift mit seinen Auslegern schließlich die Rastmittel 311, 310 und verriegelt diese in ihrer Eingriffsstellung. Das Entriegeln wird durch Verstellen der Drehachse 306 und damit des Nockens 307 bewirkt, der die Riegel 309 freigibt, die durch die Feder 309 in die Entriegelungsstellung bewegt werden, wie sie in Fig. 12 gezeigt ist.

## Patentansprüche

1. Messvorrichtung (1) zur Bestimmung eines Oberflächenverlaufs (42) eines menschlichen oder tierischen Körpers (41), wobei die Messvorrichtung (1) eine Hauptkette (43) mit mehreren Hauptkettengliedern (44, 44', 44", 44"',44^{IV}, 44^{V}) aufweist, welche durch ein Drehgelenk (45) zwischen benachbarten Hauptkettengliedern (44, 44', 44", 44"',44^{IV}, 44^{V}) miteinander verbunden sind, wobei das Drehgelenk (45) eine Skala (13,23,74') oder Sensoren aufweist, mittels welcher der Winkel zwischen den wenigstens zwei Hauptkettengliedern (44, 44', 44", 44"',44^{IV}, 44^{V}) ablesbar oder detektierbar ist, wobei die Messvorrichtung (1) vorzugsweise wenigstens eine Nebenkette (53) mit mehreren drehbeweglich verbundenen Nebenkettengliedern (54, 54', 54", 54"', 54^{IV}, 54^{V}) aufweist, wobei die Nebenkette (53) mittels eines Drehgelenks (55) mit einem der Hauptkettenglieder (44, 44', 44", 44"',44^{IV}, 44^{V}) verbunden ist, **dadurch gekennzeichnet, dass** die Messvorrichtung (1) wenigstens ein Mittel (200) zur Bestimmung wenigstens einer Neigungsrichtung relativ zum Schwerefeld eines Hauptkettengliedes (44, 44', 44", 44"',44^{IV}, 44^{V}) oder eines Nebenkettengliedes (54, 54', 54", 54"', 54^{IV}, 54^{V}) aufweist.

2. Messvorrichtung (1) nach Anspruch 1, **dadurch gekennzeichnet, dass** die Messvorrichtung (1) wenigstens eine Nebenkette (53) mit mehreren drehbeweglich verbundenen Nebenkettengliedern (54, 54', 54", 54"', 54^{IV}, 54^{V}) aufweist, wobei die Nebenkette (53) mittels eines Drehgelenks (55) mit einem der Hauptkettenglieder (44, 44', 44", 44"',44^{IV}, 44^{V}) verbunden ist.

3. Messvorrichtung (1) nach dem vorhergehenden Anspruch, wobei die Messvorrichtung (1) eine Mehrzahl von Nebenketten (53) aufweist.

4. Messvorrichtung nach einem der beiden vorhergehenden Ansprüche, wobei die Nebenkette an einem Hauptkettenglied (44, 44', 44", 44"',44^{IV}, 44^{V}) befestigt ist, das jeweils zwei entlang einer ersten Richtung (100) gegenüberliegende erste Endabschnitte (10) aufweist, welche jeweils einen ersten Gelenkbeschlag (11) zur Befestigung mit den weiteren Hauptkettengliedern umfassen, und das Hauptkettenglied (44, 44', 44", 44"',44^{IV}, 44^{V}) ferner wenigstens einen zur ersten Richtung (100) winkelig angeordneten zweiten Endabschnitt (20) aufweist, welcher einen zweiten Gelenkbeschlag (21) zur Befestigung der Nebenkette (53) umfasst.

5. Messvorrichtung (1) nach einem der vorhergehenden Ansprüche, wobei das Mittel (200) zur Bestimmung wenigstens einer Neigungsrichtung eines Hauptkettengliedes (44, 44', 44", 44"',44^{IV}, 44^{V}) oder eines Nebenkettengliedes (54) eine Wasserwaagenlibelle (211) und eine Skala (213) aufweist.

6. Messvorrichtung (1) nach einem der vorhergehenden Ansprüche, wobei eine das jeweilige Kettenglied identifizierende Markierung, beispielsweise Nummerierung, auf jedem Haupt- (44, 44', 44", 44"',44^{IV}, 44^{V}) und/oder Nebenkettenglied (54, 54', 54", 54"', 54^{IV}, 54^{V}) vorgesehen ist.

7. Messvorrichtung (1) nach einem der vorhergehenden Ansprüche, wobei das Drehgelenk (45, 55) Rastmittel, insbesondere Formschlussmittel (14, 24, 34, 201, 203, 310, 311), zur jeweiligen Verrastung zweier benachbarter Hauptkettenglieder (44, 44', 44", 44"',44^{IV}, 44^{V}), zweier benachbarter Nebenkettenglieder (54, 54', 54", 54"', 54^{IV}, 54^{V}) und/oder eines benachbarten Haupt- und Nebenkettengliedes (44, 44', 44", 44"',44^{IV}, 44^{V}, 54, 54', 54", 54"', 54^{IV}, 54^{V}) in der jeweiligen Winkellage umfasst.

8. Messvorrichtung (1) nach einem der vorhergehenden Ansprüche wobei das Drehgelenk (45, 55) Reibschlussmittel (71, 72, 73) zur jeweiligen reibschlüssigen Fixierung zweier benachbarter Hauptkettenglieder (44, 44', 44", 44"', 44^{IV}, 44^{V}), zweier benachbarter Nebenkettenglieder (54, 54', 54", 54"', 54^{IV}, 54^{V}) und/oder eines benachbarten Haupt- und Nebenkettengliedes (44, 44', 44", 44"', 44^{IV}, 44^{V}, 54, 54', 54", 54"', 54^{IV}, 54^{V}) in der jeweiligen Winkellage umfasst.

9. Messvorrichtung (1) nach einem der vorhergehenden Ansprüche, die Verriegelungsmittel (306, 307, 308) zur jeweiligen Fixierung zweier benachbarter Hauptkettenglieder (44, 44', 44", 44"',44^{IV}, 44^{V}), zweier benachbarter Nebenkettenglieder (54, 54', 54", 54"', 54^{IV}, 54^{V}) und/oder eines benachbarten Haupt- und Nebenkettengliedes (44, 44', 44", 44"',44^{IV}, 44^{V}, 54, 54', 54", 54"', 54^{IV}, 54^{V}) in der jeweiligen Winkellage umfasst.

10. Messvorrichtung (1) nach einem der vorhergehenden Ansprüche, wobei eine einstellbare Längsverstellung (38) wenigstens eines Haupt- (44, 44', 44", 44"',44^{IV}, 44^{V}) oder Nebenkettengliedes (54, 54', 54", 54"', 54^{IV}, 54^{V}) vorgesehen ist.

11. Messvorrichtung (1) nach dem vorhergehenden Anspruch, wobei die Längsverstellung (38) ein Langloch (37) und eine entlang des Langlochs (37) angeordnete weitere Skala (33) aufweist.

12. Verfahren zur Bestimmung eines Oberflächenverlaufs eines menschlichen oder tierischen Körpers, insbesondere eines Pferderückens (41'), mit einer Messvorrichtung (1) nach einem der vorhergehenden Ansprüche, wobei im ersten Verfahrensschritt die Messvorrichtung (1) auf der Oberfläche (41') angeordnet wird und die Messvorrichtung (1) an den Oberflächenverlauf (42) angepasst wird und wobei in einem zweiten Verfahrensschritt Skalenwerte oder Sensoren der Messvorrichtung (1) abgelesen oder ausgelesen werden **dadurch gekennzeichnet, dass** im zweiten Verfahrensschritt wenigstens eine Neigungsrichtung relativ zum Schwerefeld eines Haupt- oder Nebenkettengliedes, bevorzugt eines Hauptkettengliedes, bestimmt wird.

13. Verwendung der Messvorrichtung (1) nach einem der Ansprüche 1 bis 11 zur Herstellung, Anpassung und/oder Kontrolle einer Pferdeausrüstung insbesondere eines Sattels oder Sattelbockes.

## Claims

1. A measuring device (1) for determining a surface contour (42) of a human or animal body (41), the measuring device (1) comprising a main chain (43) with a plurality of main-chain links (44, 44', 44", 44"',44^{IV}, 44^{V}) which are interconnected by a hinge joint (45) between adjacent main-chain links (44, 44', 44", 44"',44^{IV}, 44^{V}), wherein the hinge joint (45) comprises a scale (13, 23, 74') or sensors, with which the angle between the at least two main-chain links (44, 44', 44", 44'",44^{IV}, 44^{V}) can be read off or detected, wherein the measuring device (1) preferably comprises at least one side chain (53) with several rotatably connected side-chain links (54, 54', 54", 54"', 54^{IV}, 54^{V}), the side chain (53) being connected to one of the main-chain links (44, 44', 44", 44"',44^{IV}, 44^{V}) by means of a hinge joint (55), **characterized in that** the measuring device (1) comprises at least one means (200) for determining at least one direction of inclination relative to the field of gravity of a main-chain link (44, 44', 44", 44"',44^{IV}, 44^{V}) or of a side-chain link (54, 54', 54", 54'", 54^{IV}, 54^{V}).

2. The measuring device (1) according to claim 1, **characterized in that** the measuring device (1) comprises at least one side chain (53) with several rotatably connected side-chain links (54, 54', 54", 54"', 54^{IV}, 54^{V}), the side chain (53) being connected to one of the main-chain links (44, 44', 44", 44"',44^{IV}, 44^{V}) by means of a hinge joint (55).

3. The measuring device (1) according to the preceding claim, wherein the measuring device (1) comprises a plurality of side chains (53).

4. The measuring device according to any one of the two preceding claims, wherein the side chain is attached to a main-chain link (44, 44', 44", 44"',44^{IV}, 44^{V}) which respectively has two first end portions (10) opposite along a first direction (100), each of which comprises a first joint fitting (11) for attachment to the further main-chain links, and the main-chain link (44, 44', 44", 44"',44^{IV}, 44^{V}) further comprises at least one second end portion (20) disposed at an angle to the first direction (100), which comprises a second joint fitting (21) for attachment of the side chain (53).

5. The measuring device (1) according to any one of the preceding claims, wherein the means (200) for determining at least one direction of inclination of a main-chain link (44, 44', 44", 44"',44^{IV}, 44^{V}) or of a side-chain link (54) comprises a bubble level (211) and a scale (213).

6. The measuring device (1) according to any one of the preceding claims, wherein a marker identifying the respective chain link, for example a numbering, is provided on each main-chain link (44, 44', 44", 44"',44^{IV}, 44^{V}) and/or side-chain link (54, 54', 54", 54"', 54^{IV}, 54^{V}).

7. The measuring device (1) according to any one of the preceding claims, wherein the hinge joint (45, 55) comprises latching means, in particular positive-engagement means (14, 24, 34, 201, 203, 310, 311), for respectively latching two adjacent main-chain links (44, 44', 44", 44"',44^{IV}, 44^{V}), two adjacent side-chain links (54, 54', 54", 54"', 54^{IV}, 54^{V}), and/or an adjacent main-chain and side-chain link (44, 44', 44", 44"',44^{IV}, 44^{V}, 54, 54', 54", 54"', 54^{IV}, 54^{V}) in the respective angular position.

8. The measuring device (1) according to any one of the preceding claims, wherein the hinge joint (45, 55) comprises friction-engagement means (71, 72, 73) for respectively fixing by frictional engagement two adjacent main-chain links (44, 44', 44", 44"',44^{IV}, 44^{V}), two adjacent side-chain links (54, 54', 54", 54"', 54^{IV}, 54^{V}), and/or an adjacent main-chain and side-chain link (44, 44', 44", 44"',44^{IV}, 44^{V}, 54, 54', 54", 54"', 54^{IV}, 54^{V}) in the respective angular position.

9. The measuring device (1) according to any one of the preceding claims, wherein locking means (306, 307, 308) are provided for respectively fixing two adjacent main-chain links (44, 44', 44", 44"',44^{IV}, 44^{V}), two adjacent side-chain links (54, 54', 54", 54"', 54^{IV}, 54^{V}), and/or an adjacent main-chain and side-chain link (44, 44', 44", 44"',44^{IV}, 44^{V}, 54, 54', 54", 54"', 54^{IV}, 54^{V}) in the respective angular position.

10. The measuring device (1) according to any one of the preceding claims, wherein an adjustable longitudinal adjustment means (38) of at least one main-chain link (44, 44', 44", 44"',44^{IV}, 44^{V}) or side-chain link (54, 54', 54", 54"', 54^{IV}, 54^{V}) is provided.

11. The measuring device (1) according to the preceding claim, wherein the longitudinal adjustment means (38) comprises an elongated hole (37) and a further scale (33) disposed along the elongated hole (37).

12. A method for determining a surface contour of a human or animal body, in particular of a horse's back (41'), with a measuring device (1) according to any one of the preceding claims, wherein, in the first method step, the measuring device (1) is disposed on the surface (41') and the measuring device (1) is adapted to the surface contour (42), and wherein, in a second method step, scale values or sensors of the measuring device (1) are read off or read out, **characterized in that** at least one direction of inclination relative to the field of gravity of a main-chain or side-chain link, preferably of a main-chain link, is determined in the second method step.

13. A use of the measuring device (1) according to any one of the claims 1 to 11 for the manufacture, adaptation and/or inspection of equestrian equipment, in particular a saddle or saddle rack.

## Revendications

1. Dispositif de mesure (1) destiné à déterminer une allure de surface (42) d'un corps humain ou animal (41), le dispositif de mesure (1) comprenant une chaîne principale (43) ayant une pluralité d'éléments de chaîne principale (44, 44', 44", 44"', 44^{IV}, 44^{V}) qui sont reliés entre eux par un joint rotatif (45) entre des éléments de chaîne principale (44, 44', 44", 44"', 44^{IV}, 44^{V}) voisins, dans lequel ledit joint rotatif (45) comprend une échelle (13, 23, 74') ou des capteurs à l'aide desquels il est possible de lire ou de détecter l'angle entre lesdits aux moins deux éléments de chaîne principale (44, 44', 44", 44"', 44^{IV}, 44^{V}), dans lequel ledit dispositif de mesure (1) comprend de préférence au moins une chaîne secondaire (53) ayant une pluralité d'éléments de chaîne secondaire (54, 54', 54", 54"', 54^{IV}, 54^{V}) reliés entre eux de manière mobile en rotation, dans lequel ladite chaîne secondaire (53) est reliée au moyen d'un joint rotatif (55) à l'un des éléments de chaîne principale (44, 44', 44", 44"', 44^{IV}, 44^{V}), **caractérisé par le fait que** le dispositif de mesure (1) comprend au moins un moyen (200) destiné à déterminer au moins une direction d'inclinaison par rapport au champ de pesanteur d'un élément de chaîne principale (44, 44', 44", 44"', 44^{IV}, 44^{V}) ou d'un élément de chaîne secondaire (54, 54', 54", 54"', 54^{IV}, 54^{V}).

2. Dispositif de mesure (1) selon la revendication 1, **caractérisé par le fait que** le dispositif de mesure (1) comprend au moins une chaîne secondaire (53) ayant une pluralité d'éléments de chaîne secondaire (54, 54', 54", 54"', 54^{IV}, 54^{V}) reliés entre eux de manière mobile en rotation, dans lequel ladite chaîne secondaire (53) est reliée au moyen d'un joint rotatif (55) à l'un des éléments de chaîne principale (44, 44', 44", 44"', 44^{IV}, 44^{V}).

3. Dispositif de mesure (1) selon la revendication précédente, dans lequel le dispositif de mesure (1) présente une pluralité de chaînes secondaires (53).

4. Dispositif de mesure selon l'une quelconque des deux revendications précédentes, dans lequel la chaîne secondaire est fixée sur un élément de chaîne principale (44, 44', 44", 44"', 44^{IV}, 44^{V}) qui présente respectivement deux premières portions d'extrémité (10) qui sont situées en regard l'une de l'autre le long d'une première direction (100) et qui comprennent chacune une première garniture d'articulation (11) pour la fixation avec les autres éléments de chaîne principale, et l'élément de chaîne principale (44, 44', 44", 44"', 44^{IV}, 44^{V}) présente en outre au moins une deuxième portion d'extrémité (20) qui est disposée angulairement par rapport à ladite première direction (100) et qui comprend une deuxième garniture d'articulation (21) pour la fixation de la chaîne secondaire (53).

5. Dispositif de mesure (1) selon l'une quelconque des revendications précédentes, dans lequel le moyen (200) de détermination d'au moins une direction d'inclinaison d'un élément de chaîne principale (44, 44', 44", 44"', 44^{IV}, 44^{V}) ou d'un élément de chaîne secondaire (54) présente un niveau à bulle d'air (211) et une échelle (213).

6. Dispositif de mesure (1) selon l'une quelconque des revendications précédentes, dans lequel un marquage, par exemple une numérotation, identifiant l'élément de chaîne respectif est prévu(e) sur chaque élément de chaîne principale (44, 44', 44", 44"', 44^{IV}, 44^{V}) et/ou élément de chaîne secondaire (54, 54', 54", 54"', 54^{IV}, 54^{V}).

7. Dispositif de mesure (1) selon l'une quelconque des revendications précédentes, dans lequel le joint rotatif (45, 55) comprend des moyens d'encliquetage, en particulier des moyens à engagement positif (14, 24, 34, 201, 203, 310, 311), pour l'encliquetage respectif de deux éléments voisins de chaîne principale (44, 44', 44", 44"', 44^{IV}, 44^{V}), de deux éléments voisins de chaîne secondaire (54, 54', 54", 54"', 54^{IV}, 54^{V}) et/ou d'un élément voisin de chaîne principale et de chaîne secondaire (44, 44', 44", 44"', 44^{IV}, 44^{V}, 54, 54', 54", 54"', 54^{IV}, 54^{V}) dans la position angulaire respective.

8. Dispositif de mesure (1) selon l'une quelconque des revendications précédentes, dans lequel le joint rotatif (45, 55) comprend des moyens de liaison par friction (71, 72, 73) pour la fixation respective entraînée par friction de deux éléments voisins de chaîne principale (44, 44', 44", 44"', 44^{IV}, 44^{V}), de deux éléments voisins de chaîne secondaire (54, 54', 54", 54"', 54^{IV}, 54^{V}) et/ou d'un élément voisin de chaîne principale et de chaîne secondaire (44, 44', 44", 44"', 44^{IV}, 44^{V}, 54, 54', 54", 54"', 54^{IV}, 54^{V}) dans la position angulaire respective.

9. Dispositif de mesure (1) selon l'une quelconque des revendications précédentes, dans lequel des moyens de verrouillage (306, 307, 308) pour la fixation respective de deux éléments voisins de chaîne principale (44, 44', 44", 44"', 44^{IV}, 44^{V}), de deux éléments voisins de chaîne secondaire (54, 54', 54", 54"', 54^{IV}, 54^{V}) et/ou d'un élément voisin de chaîne principale et de chaîne secondaire (44, 44', 44", 44"', 44^{IV}, 44^{V}, 54, 54', 54", 54"', 54^{IV}, 54^{V}) dans la position angulaire respective sont prévus.

10. Dispositif de mesure (1) selon l'une quelconque des revendications précédentes, dans lequel un ajustage longitudinal (38) réglable d'au moins un élément de chaîne principale (44, 44', 44", 44"', 44^{IV}, 44^{V}) ou un élément de chaîne secondaire (54, 54', 54", 54"', 54^{IV}, 54^{V}) est prévu.

11. Dispositif de mesure (1) selon la revendication précédente, dans lequel l'ajustage longitudinal (38) présente un trou oblong (37) et une autre échelle (33) disposée le long du trou oblong (37).

12. Procédé de détermination d'une allure de surface d'un corps humain ou animal, en particulier d'un dos de cheval (41'), au moyen d'un dispositif de mesure (1) selon l'une quelconque des revendications précédentes, dans lequel, dans la première étape de procédé, ledit dispositif de mesure (1) est disposé sur la surface (41') et le dispositif de mesure (1) est adapté à l'allure de surface (42), et dans lequel, dans une deuxième étape de procédé, des valeurs d'échelle ou des capteurs du dispositif de mesure (1) sont lus ou extraits, **caractérisé par le fait que**, dans la deuxième étape de procédé, au moins une direction d'inclinaison par rapport au champ de pesanteur d'un élément de chaîne principale ou de chaîne secondaire, de préférence d'un élément de chaîne principale est déterminée.

13. Utilisation du dispositif de mesure (1) selon l'une quelconque des revendications 1 à 11, pour la fabrication, l'adaptation et/ou le contrôle d'un équipement de cheval, en particulier d'une selle ou d'un chevalet de selle.
